# EUROPEAN PATENT SPECIFICATION

(11) **EP 4 547 642 B1**
(45) Date of publication and mention of the grant of the patent: **03.06.2026**
(21) Application number: 23735724.9
(22) Date of filing: 27.06.2023
(51) Int. Cl.: C07C 201/08, C07C 205/06

(54) **PROCESS FOR PRODUCING NITROBENZENE**
VERFAHREN ZUM PRODUZIEREN VON NITROBENZOL
PROCÉDÉ DE PRODUCTION DE NITROBENZÈNE

(30) Priority: 28.06.2022 EP 22181580
(43) Date of publication of application: 07.05.2025
(73) Proprietor: BASF SE, 67056 Ludwigshafen am Rhein (DE)
(72) Inventor: BLANKERS, Bart, 2040 Antwerpen (BE); VERCRUYSSE, Karen, 2040 Antwerpen (BE); DEBERDT, Filip, 2040 Antwerpen (BE); BAERT, Jonathan, 6811 Arnhem (NL); DE WINNE, Hendrik, 2040 Antwerpen (BE)
(74) Representative: BASF IP Association
(86) International application number: PCT/EP2023/067478
(87) International publication number: WO 2024/003050

(56) References cited:
- CN-A- 112 939 781
- US-A1- 2009 187 051
- US-A1- 2016 083 332

## Description

The invention relates to a process for producing nitrobenzene by an adiabatic nitration of benzene and nitric acid in the presence of sulfuric acid, the process comprising:
(a) feeding benzene, nitric acid and sulfuric acid into a reaction zone;
(b) producing a reaction mixture comprising water, nitrobenzene and sulfuric acid by reaction of the benzene and the nitric acid in the presence of the sulfuric acid in the reaction zone; and
(c) separating the reaction mixture into an aqueous phase comprising water and sulfuric acid and an organic phase comprising crude nitrobenzene by phase separation.

Producing nitrobenzene by an adiabatic nitration of benzene with nitric acid in the presence of sulfuric acid is a well-known process and described for example in DE-A 10 2017 110084, WO-A 2014/177450 or in EP-B 2 070 907.

One disadvantage of the known processes is the formation of black precipitates. These black precipitates are described for example in US-A 2016/0083332 and CN-A 112939781 and are found in the apparatus for phase separation in which the crude nitrobenzene is separated from the aqueous phase comprising sulfuric acid and water or in heat exchangers used to cool down the crude nitrobenzene which is discharged from the apparatus for phase separation. Further, if the reaction is carried out in such a way that the reaction mixture is leaving the reactor as gas, the black precipitates also are found in a condenser for the reaction mixture and in pipelines connecting the several apparatuses of the plant for producing the nitrobenzene.

According to US-A 2016/0083332 and CN-A 112939781, an oxidizing or reducing agent is added to the circulating sulfuric acid. However, adding the oxidizing or reducing agent requires the installation of additional apparatuses in the plant to store and add the oxidizing/reducing agent and therefore increases overall installation cost and operating complexity.

Therefore, it is an object of the present invention to provide a process for producing nitrobenzene by an adiabatic nitration of benzene and nitric acid in the presence of sulfuric acid where the formation of the black precipitates is reduced or prevented without the requirement to add additional apparatuses to the plant.

This object is achieved by a process for producing nitrobenzene by an adiabatic nitration of benzene and nitric acid in the presence of sulfuric acid, the process comprising:
(a) feeding benzene, nitric acid and sulfuric acid into a reaction zone;
(b) producing a reaction mixture comprising water, nitrobenzene and sulfuric acid by reaction of the benzene and the nitric acid in the presence of the sulfuric acid in the reaction zone; and
(c) separating the reaction mixture into an aqueous phase comprising water and sulfuric acid and an organic phase comprising crude nitrobenzene by phase separation;
wherein the concentration of titanium in the reaction mixture is kept below 170 mg/kg.

In the context of the present invention, the concentration unit "mg/kg" refers to milligrams titanium per kilogram liquid, wherein the mass of the liquid includes all components which are contained in the liquid including dissolved and/or dispersed solids like salts. The titanium thereby may be present in the liquid as metallic particles, as titanium ions, or as metallic particles and ions.

Surprisingly it has shown that the black precipitates can be reduced or even avoided when the concentration of titanium in the reaction mixture is kept below 170 mg/kg. More preferred, the concentration of titanium in the reaction mixture is kept below 85 mg/kg and particularly below 25 mg/kg.

Keeping the concentration of titanium in the reaction mixture below 170 mg/kg can be achieved by any suitable measure known to a skilled person, for example by feeding nitric acid having a concentration of titanium below 1 mg/kg or by entrainment of at least 1.8 g H₂SO₄ per kilogram nitrobenzene into the organic phase in the phase separation in step (c).

As the titanium enters the process particularly as an impurity in the nitric acid fed into the process, it is preferred to feed nitric acid having a concentration of titanium below 1 mg/kg, more preferred below 0.5 mg/kg and particularly below 0.1 mg/kg. The titanium enters the nitric acid particularly during the production of nitric acid, for example through chemical reaction, such as corrosion of plant equipment with which the nitric acid comes into contact. The rate of the chemical reaction is strongly influenced by temperature. The higher the temperature is, the faster is the rate of reaction. For a nitric acid plant and storage tanks, a higher rate of reaction means a higher corrosion rate and, thus, a higher concentration of titanium in the nitric acid. It is therefore advantageous to cool down the nitric acid as quickly as possible after production and to store the nitric acid sufficiently cold in tanks to limit the chemical reactions with plant equipment, particularly corrosion.

The temperature to which the nitric acid is cooled after production and the storage temperature thereby depend on the production process for the nitric acid and the material, the plant equipment is made of.

Besides reducing the amount of titanium entering the nitric acid during the production process or during storage by cooling and storing the nitric acid at a sufficiently low temperature to decelerate the reaction rate and thus the corrosion by which titanium may enter the nitric acid, it is also possible to work up the nitric acid after production to reduce the concentration of the titanium in the nitric acid.

However, independently on how the concentration of titanium is set in the nitric acid, according to a first alternative of the invention, it is important that the concentration of titanium in the nitric acid fed into the production process for nitrobenzene is below 1 mg/kg.

It is the current state of art to reconcentrate and recycle the sulfuric acid used in the production process of the nitrobenzene into the reaction. If the sulfuric acid is recycled, the titanium usually concentrates in the aqueous phase. Therefore, to avoid an accumulation of titanium until it reaches a concentration in the reaction mixture of more than 170 mg/kg, more preferred of more than 85 mg/kg and particularly of more than 25 mg/kg, it is necessary to limit the amount of titanium in the sulfuric acid being recycled into the reaction.

This limitation of the concentration of titanium in the sulfuric acid being recycled into the reaction and thus keeping the concentration of titanium in the reaction mixture below 170 mg/kg, may be achieved for example by entrainment of aqueous phase into the organic phase. Preferably, an amount of the aqueous phase is entrained into the organic phase which is such that at least 1.8 g H₂SO₄ per kilogram nitrobenzene is entrained into the organic phase. More preferred, the amount of aqueous phase being entrained into the organic phase is such that at least 3.6 g H₂SO₄ per kilogram nitrobenzene are entrained into the organic phase. This can be achieved by, for example, limiting the size of the phase separator to decrease the available separation time.

This entrainment of H₂SO₄ into the organic phase is in contrast to present processes, where the entrainment of H₂SO₄ into the organic phase is limited, as this entrainment leads to an increase in sulfates in the waste water, an increased addition of fresh sulfuric acid, and, in case the waste water is neutralized, a higher consumption of neutralization agent like caustic soda. However, these disadvantages are less significant than the formation of the black precipitates, which requires regular shut-downs of the process for cleaning the apparatuses and, thus, results in a loss of plant capacity due to lower availability.

On the other hand, if the concentration of titanium in the reaction mixture below 170 mg/kg is achieved by another suitable measure, for example by feeding nitric acid having a concentration of titanium below 1 mg/kg and not by entrainment of sulfuric acid into the organic phase, the amount of fresh sulfuric acid fed into the process may be kept at such a low level as in present processes.

"Per kilogram nitrobenzene" in this context means the amount of pure nitrobenzene in the organic phase. Thus, the amount of the organic phase usually is larger, as the organic phase additionally may contain organic impurities, by-products obtained in the reaction and further the entrained aqueous phase. This means that the whole amount of aqueous phase being entrained into the organic phase during phase separation depends on the concentration of the sulfuric acid and usually is higher than the above specified amount of H₂SO₄ because besides the H₂SO₄ also water and further impurities being solved in the water are entrained into the organic phase. The amount of the H₂SO₄ entrained into the organic phase in the phase separation preferably is selected such that the concentration of titanium in the concentrated sulfuric acid recycled into the reaction zone is at a level to ensure the titanium present in the reaction mixture is kept below 170 mg/kg, more preferred below 85 mg/kg and particularly below 30 mg/kg.

By entrainment of H₂SO₄ containing aqueous phase, in the following also denoted as sulfuric acid or aqueous sulfuric acid, into the organic phase in the phase separation, also titanium being contained in the aqueous sulfuric acid is entrained into the organic phase. As the sulfuric acid being entrained into the organic phase is withdrawn from the process, also the titanium being entrained into the organic phase by this entrainment is withdrawn. For having a sufficient amount of sulfuric acid in the reaction, the sulfuric acid being entrained into the organic phase is replaced by fresh sulfuric acid. As fresh sulfuric acid usually does not contain titanium as an impurity, no additional titanium is fed into the process by means of the fresh sulfuric acid.

The sulfuric acid usually is not consumed during the reaction and can therefore be returned into the process. However, as water forms in the reaction, the sulfuric acid obtained as aqueous phase by phase separation of the reaction mixture is diluted. For this reason, if it is intended to reuse the sulfuric acid, the water must be removed from the aqueous phase to concentrate the sulfuric acid, before recycling the sulfuric acid into the reaction.

Removing water from the aqueous phase for concentrating the sulfuric acid can be carried out by any process known to a skilled person. Such a process is described for example in WO-A 2014/177450 or US 4,091,042. Usually, the sulfuric acid is concentrated by flash evaporation to remove the water in preferably one evaporation step. The concentration to which the sulfuric acid is concentrated preferably corresponds to the concentration with which the sulfuric acid is fed into the reaction zone and which preferably is in a range from 60 to 96 wt%.

The thus obtained concentrated sulfuric acid then can be recycled into the reaction. For recycling, the sulfuric acid preferably is mixed with nitric acid and then fed into the reaction zone.

The reaction zone may be any suitable reactor in which the nitration can be carried out. The nitration can be carried out batchwise or continuously, wherein a continuous process is preferred. The reactor used for producing the nitrobenzene may be for example a stirred tank reactor or a cascade of stirred tank reactors, a loop reactor or a tubular reactor. Preferably, the reactor is a tubular reactor and particularly preferably, the reactor is a tubular reactor having dispersion elements as described for example in US 4,994,242, US-A 2003/0055300, EP-A 1 272 268 or DE-A 10 2017 110 084.

The temperature at which the reaction is carried out, preferably is in a range from 70 to 145 °C. The nitric acid fed into the reaction zone preferably has a concentration in a range from 60 to 98 wt% and the sulfuric acid a concentration in the range from 60 to 96 wt%. Benzene preferably is fed in a stoichiometric amount, however particularly preferably, benzene is fed in an extent of 2 to 10 % compared to the stoichiometrically required amount.

The reaction mixture, obtained in the reaction zone, is fed into an apparatus for phase separation to separate the mixture into the organic phase comprising crude nitrobenzene and the aqueous phase comprising water and sulfuric acid. As the reaction, the phase separation may be carried out either batchwise or continuously, a continuous phase separation being preferred. The phase separation apparatus used for separating the organic phase containing crude nitrobenzene and the aqueous phase containing water and sulfuric acid can be any phase separation apparatus known to a skilled person.

The organic phase containing the crude nitrobenzene then is further worked up to obtain nitrobenzene having a higher purity as a product. Particularly preferably, by working up the organic phase containing the crude nitrobenzene, nitrobenzene having a purity of 99 to 99.99 wt % is obtained. Working up the organic phase usually comprises washing by adding an aqueous phase to remove dissolved and suspended acids, followed by a further phase separation into an organic phase comprising nitrobenzene and an aqueous phase comprising a sulfate and titanium. The aqueous phase obtained in the phase separation is withdrawn from the process as waste water and usually fed into a waste water purification treatment.

The organic phase comprising nitrobenzene obtained in the phase separation optionally may be subject to further purification steps like extraction and distillation steps for removing low boilers and high boilers.

If the concentration of titanium in the reaction mixture is kept below 170 mg/kg by using nitric acid having a concentration of titanium below 1 mg/kg, preferably below 0.5 mg/kg and particularly below 0.1 mg/kg, the phase separation of the reaction mixture may be carried out as in the known processes. However, also if such a nitric acid is used, it is further possible to additionally entrain sulfuric acid into the organic phase in an amount of at least 1.8 g H₂SO₄ per kilogram nitrobenzene.

An embodiment of the invention is shown in the figure and explained in more detail in the following description.

In the figures:

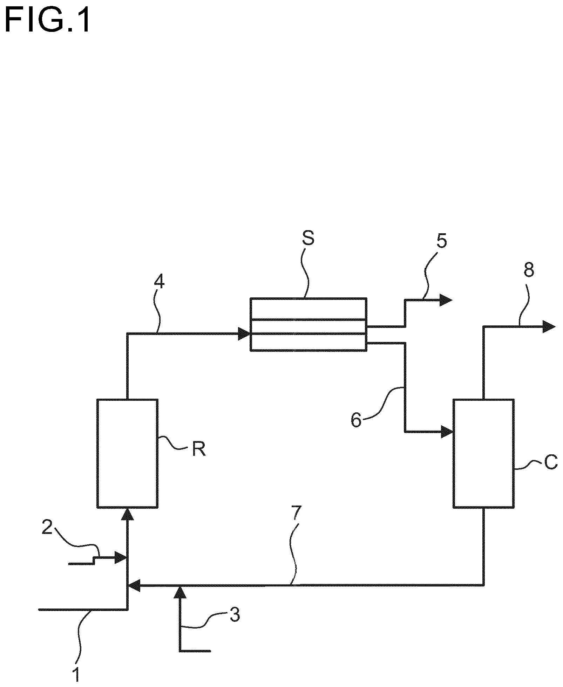

- Figure 1: shows a flow chart of a process for adiabatic nitration of benzene and nitric acid in the presence of sulfuric acid;

The only figure shows schematically a process for adiabatic nitration of benzene and nitric acid.

For producing nitrobenzene, nitric acid 1 having a concentration in a range from 60 to 98 wt% is mixed with sulfuric acid 7, having a concentration in a range from 60 to 96 wt%. After mixing the nitric acid and the sulfuric acid, benzene 2 is added to this mixture. The benzene 2 preferably is added in a stoichiometric amount or in excess, preferably in an excess of 2 to 10 % compared to the stoichiometric amount.

The mixture of nitric acid, sulfuric acid and benzene is fed into a reactor R. In the reactor R, the nitric acid and the benzene react in the presence of the sulfuric acid, forming nitrobenzene and water. The reaction is exothermic and as the reaction is carried out adiabatically, the temperature increases and the reaction mixture comprising nitrobenzene, water and sulfuric acid has a higher temperature than the mixture of nitric acid, sulfuric acid and benzene fed into the reactor. For carrying out the reaction adiabatically, the reactor is thermally insulated to minimize reaction heat being dissipated to the environment.

The reaction mixture 4 obtained in the reactor is fed into a phase separator S. In the phase separator S, the reaction mixture 4 is separated into an organic phase 5 which comprises crude nitrobenzene and an aqueous phase 6 comprising the sulfuric acid.

The organic phase 5 is further worked up for purification of nitrobenzene. For this purpose, the organic phase which still contains small amounts of sulfuric acid is washed with water and subsequently subjected to a further phase separation in which the aqueous phase which comprises sulfates is separated from the organic phase comprising the nitrobenzene. The organic phase then may be further purified, for example by extraction and distillation to remove low boilers and high boilers to obtain pure nitrobenzene. The whole purification process is carried out as known by the skilled person.

The aqueous phase obtained by the phase separation in the phase separator S is fed into a concentrator C to reconcentrate the sulfuric acid by removing water from the aqueous phase. The concentrator C may comprise one or more than one stage for reconcentrating the sulfuric acid. The water preferably is removed from the aqueous phase by evaporation for concentrating the sulfuric acid. For this purpose, the concentrator C may comprise at least one evaporator in which the water is evaporated and removed as vapor 8. The vapor 8 then is condensed and withdrawn from the process as waste water and subjected to a waste water purification before being released to the environment.

The reconcentrated sulfuric acid 7 obtained in the concentrator C then is recycled into the reaction and mixed with the nitric acid 1. As some of the sulfuric acid is entrained into the organic phase in the phase separator S, fresh sulfuric acid 3 is added to the recycled sulfuric acid 7 before being mixed with the nitric acid 1. However, besides adding the fresh sulfuric acid 3 with the recycled, reconcentrated sulfuric acid 7 before mixing with the nitric acid, it is also possible to mix the recycled sulfuric acid 7, the fresh sulfuric acid 3 and the nitric acid 1 simultaneously.

To minimize or preferably avoid formation of black precipitates, according to the invention the amount of titanium in the reaction mixture is kept below 170 mg/kg, more preferred below 85 mg/kg and particularly below 30 mg/kg. To achieve these concentrations in one embodiment of the invention nitric acid 1 having a concentration of titanium of less than 1 mg/kg, more preferred of less than 0.5 mg/kg and particularly below 0.1 mg/kg, is added into the process.

In an alternative embodiment, the amount of sulfuric acid entrained into the organic phase in the phase separator S is at least 1.8 g H₂SO₄ per kilogram nitrobenzene and particularly at least 3.6 g H₂SO₄ per kilogram nitrobenzene. This small amount of sulfuric acid being entrained into the organic phase in the phase separator S is sufficient to achieve the intended concentration of titanium in the reaction mixture and to minimize or even avoid formation of black precipitates.

Besides using nitric acid having a concentration of titanium below 1 mg/kg or entrain more than 1.8 g H₂SO₄ per kilogram nitrobenzene as alternatives to keep the concentration of titanium in the reaction mixture below 170 mg/kg, it is also possible to use nitric acid having a concentration of titanium of less than 1 mg/kg, more preferred of less than 0.5 mg/kg and particularly below 0.1 mg/kg and to entrain at least 1.8 g H₂SO₄ per kilogram nitrobenzene and particularly at least 3.6 g H₂SO₄ per kilogram nitrobenzene into the organic phase in the phase separator S.

### Examples

### Comparative Example

24.1 t/h nitric acid, 371 t/h sulfuric acid and 22.4 t/h benzene were fed into a reactor for adiabatic nitration to produce nitrobenzene. The resulting reaction mixture containing was fed into a phase separator in which 34.1 t/h of an organic phase containing nitrobenzene and 383.4 t/h of an aqueous phase containing water and sulfuric acid were obtained. Water was evaporated from the aqueous phase and the thus reconcentrated sulfuric acid was recycled into the reactor.

The amount of recycled sulfuric acid was 371 t/h and contained 400 mg/kg titanium. In the phase separator only 0.9 g H₂SO₄ per kilogram nitrobenzene were entrained.

The process showed a significant formation of black precipitates, requiring frequent stopping of the plant for cleaning.

### Example 1

The production plant for producing nitrobenzene was operated at the same operating conditions as in Comparative Example with the difference that a phase separator was used in which a larger amount of sulfuric acid was entrained into the organic phase. The amount of sulfuric acid being entrained into the organic phase was 3.6 g H₂SO₄ per kilogram nitrobenzene.

The concentration of titanium in the sulfuric acid being recycled into the reactor was below 50 mg/kg. Further, the amount of black material formed was much smaller than in the Comparative example.

### Example 2

The production plant for producing nitrobenzene was operated at the same conditions as in the Comparative Example and Example 1 but this time, the concentration of titanium in the nitric acid fed into the production process for nitrobenzene was below 0.1 mg/kg.

The concentration of titanium in the sulfuric acid being recycled into the reaction was 100 mg/kg. This amount of titanium showed a significantly lower formation of black precipitates than in the Comparative Example but was still larger than in Example 1.

## Claims

1. A process for producing nitrobenzene by an adiabatic nitration of benzene (2) and nitric acid (1) in the presence of sulfuric acid (7), the process comprising:
(a) feeding benzene (2), nitric acid (1) and sulfuric acid (7) into a reaction zone (R);
(b) producing a reaction mixture (4) comprising water, nitrobenzene and sulfuric acid by reaction of the benzene and the nitric acid in the presence of the sulfuric acid in the reaction zone (R); and
(c) separating the reaction mixture into an aqueous phase (6) comprising water and sulfuric acid and an organic phase (5) comprising crude nitrobenzene by phase separation;
wherein the concentration of titanium in the reaction mixture (4) is kept below 170 mg/kg.

2. The process according to claim 1, wherein the concentration of titanium in the reaction mixture (4) is kept below 170 mg/kg by feeding nitric acid (1) having a concentration of titanium below 1 mg/kg.

3. The process according to claim 1 or 2, wherein the concentration of titanium in the reaction mixture (4) is kept below 170 mg/kg by entrainment of at least 1.8 g H₂SO₄ per kilogram nitrobenzene into the organic phase in the phase separation in step (c).

4. The process according to any of claims 1 to 3, wherein water from the aqueous phase (6) comprising water and sulfuric acid is removed to obtain concentrated sulfuric acid.

5. The process according to claim 4, wherein the concentrated sulfuric acid (7) is recycled into the reaction zone.

6. The process according to claim 5, wherein the concentration of titanium in the concentrated sulfuric acid (7) recycled into the reaction zone is below 200 mg/kg.

7. The process according to any of claims 1 to 6, wherein the crude nitrobenzene (5) is worked up by washing with an aqueous phase and phase separation into an organic phase comprising nitrobenzene and an aqueous phase comprising a sulfate and titanium.

## Patentansprüche

1. Verfahren zur Herstellung von Nitrobenzol durch eine adiabatische Nitrierung von Benzol (2) und Salpetersäure (1) in Gegenwart von Schwefelsäure (7), wobei das Verfahren umfasst:
(a) Zuführen von Benzol (2), Salpetersäure (1) und Schwefelsäure (7) in eine Reaktionszone (R);
(b) Erzeugen eines Reaktionsgemisches (4), umfassend Wasser, Nitrobenzol und Schwefelsäure, durch Reaktion des Benzols und der Salpetersäure in Gegenwart der Schwefelsäure in der Reaktionszone (R); und
(c) Trennen des Reaktionsgemisches durch Phasentrennung in eine wässrige Phase (6), umfassend Wasser und Schwefelsäure, und eine organische Phase (5), umfassend Roh-Nitrobenzol;
wobei die Konzentration von Titan im Reaktionsgemisch (4) unter 170 mg/kg gehalten wird.

2. Verfahren nach Anspruch 1, wobei die Konzentration von Titan im Reaktionsgemisch (4) unter 170 mg/kg gehalten wird, indem Salpetersäure (1) zugeführt wird, deren Titankonzentration unter 1 mg/kg liegt.

3. Verfahren nach Anspruch 1 oder 2, wobei die Konzentration von Titan im Reaktionsgemisch (4) unter 170 mg/kg gehalten wird durch Mitnahme von mindestens 1,8 g H₂SO₄ pro Kilogramm Nitrobenzol in die organische Phase bei der Phasentrennung in Schritt (c).

4. Verfahren nach einem der Ansprüche 1 bis 3, wobei aus der wässrigen Phase (6), umfassend Wasser und Schwefelsäure, Wasser entfernt wird, um konzentrierte Schwefelsäure zu erhalten.

5. Verfahren nach Anspruch 4, wobei die konzentrierte Schwefelsäure (7) in die Reaktionszone zurückgeführt wird.

6. Verfahren nach Anspruch 5, wobei die Konzentration von Titan in der in die Reaktionszone zurückgeführten konzentrierten Schwefelsäure (7) unter 200 mg/kg liegt.

7. Verfahren nach einem der Ansprüche 1 bis 6, wobei das Roh-Nitrobenzol (5) durch Waschen mit einer wässrigen Phase und anschließende Phasentrennung in eine organische Phase, umfassend Nitrobenzol, und eine wässrige Phase, umfassend ein Sulfat und Titan, aufgearbeitet wird.

## Revendications

1. Un procédé de production de nitrobenzène par une nitration adiabatique de benzène (2) et d'acide nitrique (1) en présence d'acide sulfurique (7), le procédé comprenant :
(a) alimentant benzène (2), acide nitrique (1) et acide sulfurique (7) dans une zone de réaction (R) ;
(b) produisant un mélange réactionnel (4) composé d'eau, de nitrobenzène et d'acide sulfurique par réaction du benzène et de l'acide nitrique en présence de l'acide sulfurique dans le
zone de réaction (R) ; et
(c) séparer le mélange de réaction en une phase aqueuse (6) composée d'eau et d'acide sulfurique et d'une phase organique (5) composée de nitrobenzène brut par séparation de phase ;
où la concentration de titane dans le mélange de réaction (4) est maintenue en dessous de 170 mg/kg.

2. Le procédé, selon la revendication 1, consiste à maintenir la concentration de titane dans le mélange réactionnel (4) en dessous de 170 mg/kg en alimentant de l'acide nitrique (1) ayant une concentration de tita-Néum en dessous de 1 mg/kg.

3. Le procédé, selon la revendication 1 ou 2, consiste à maintenir la concentration de titane dans le mélange de réaction (4) en dessous de 170 mg/kg par entraînement d'au moins 1,8 g de H2SO4 par kilogramme de ni-trovenzène dans la phase organique lors de la séparation de phase à l'étape (c).

4. Le procédé selon l'une des revendications 1 à 3, dans lequel l'eau de la phase aqueuse (6) composée d'eau et d'acide sulfurique est retirée pour obtenir de l'acide sulfurique concentré.

5. Le procédé selon la revendication 4, dans lequel l'acide sulfurique concentré (7) est recyclé en la zone de réaction.

6. Le procédé, selon l'affirmation 5, consiste à obtenir la concentration de titane dans l'acide sulfurique concentré (7) recyclé dans la zone de réaction inférieure à 200 mg/kg.

7. Le procédé, selon l'une des revendications 1 à 6, consiste à exploiter le nitrobenzène brut (5) par lavage avec une phase aqueuse et une séparation de phase en une phase organique composée de nitrobenzène et une phase aqueuse comprenant un sulfate et du titane.
